# EUROPÄISCHE PATENTSCHRIFT

(11) **EP 0 441 171 B1**
(45) Veröffentlichungstag und Bekanntmachung des Hinweises auf die Patenterteilung: **05.04.1995**
(21) Anmeldenummer: 91100785.4
(22) Anmeldetag: 23.01.1991
(51) Int. Cl.: A61M 39/02

(54) **Medizinisches Gerät mit Keimsperre**
Medical device with germ barrier
Dispositif médical avec barrière à germe

(30) Priorität: 08.02.1990 DE 4003705
(43) Veröffentlichungstag der Anmeldung: 14.08.1991
(73) Patentinhaber: WEX, Roland, D-34212 Melsungen (DE)
(72) Erfinder: Steffens, Klaus-Jürgen, Prof. Dr., W-3300 Braunschweig (DE); Wex, Roland, W-3508 Melsungen (DE)
(74) Vertreter: Quermann, Helmut, Dipl.-Ing.

(56) Entgegenhaltungen:
- EP-A- 0 063 640
- DE-C- 3 515 665
- US-A- 4 666 427
- K.H. WALLHÄUSSER "Praxis der Sterilisation-Desinfektion-Konservierung", 4. Auflage, 1988, Georg Thieme Verlag Stuttgart-New York Seiten 576-591

## Beschreibung

Die Erfindung betrifft ein medizinisches Gerät zur Zuführung bzw. Entnahme von Flüssigkeiten mittels Injektion, Infusion oder Transfusion, das einen Anschluß mit einem Zuführ- bzw. Entnahmestutzen sowie ein Verschlußelement für den Stutzen aufweist, wobei das Verschlußelement mit einem keimhemmende bzw. keimtötende Eigenschaft aufweisenden Element versehen ist, das bei verschlossenem Stutzen in diesen ragt, und das Element als Stift ausgebildet ist.

Auf dem Gebiet der Medizintechnik finden derartige medizinische Geräte, beispielsweise Kanülen, Katheter, Drainagen, in vielfältigster Ausgestaltung Verwendung. Sie ermöglichen die mehrmalige Zuführung bzw. Entnahme von Flüssigkeiten, weshalb der Stutzen im Anschluß an die jeweilige Zuführung bzw. Entnahme von Flüssigkeit mittels des Verschlußelementes zu verschließen ist. Bekanntermaßen stellen die Anschlüsse bei Injektions-, Infusions- bzw. Transfusionsprodukten, wobei auch enterale Ernährungsprodukte und Urinprodukte einzubeziehen sind, eine potentielle Gefahrenstelle der Kontamination/Verkeimung bei Mehrfachbenutzung dar (siehe Studie Prof. Daschner/Freiburg "Bakterielle Probleme bei der Infusionstherapie", veröffentlicht "Klinische Pharmakologie und experimentelle Medizin", VII Kollogium Bad Kreuznach 1980).

In der Praxis hat sich gezeigt, daß im Stutzen, der in aller Regel als positiver oder negativer Stutzen ausgebildet ist, nach erfolgter Zuführung bzw. Entnahme von Flüssigkeit ein sogenannter Resttropfen zurückbleibt, der bis zur erneuten Zuführung bzw. Entnahme von Flüssigkeit verkeimen kann und damit bei einer nachfolgenden Entnahme, beispielsweise mittels einer Injektionsspritze aufgezogen, dem Patienten mittelbar bzw. bei einer Zuführung von Flüssigkeit in das Gerät dem Patienten unmittelbar zugeführt wird.

Aus der US-A-4,666,427 ist ein ausschließlich zum Zuführen von Flüssigkeiten geeignetes medizinisches Gerät der eingangs genannten Art bekannt. Dort entspricht der Außendurchmesser des Stiftes dem Innendurchmesser des Stutzens, so daß zwischen diesen kein Spalt verbleibt. Der Verschlußstopfen ist porös und insgesamt luftdurchlässig, so daß die wässrige Flüssigkeit durch diesen verdunsten kann, was beabsichtigt ist. Hierdurch werden Partikel, wie Bakterien an den Verschlußstopfen adhäriert und sterben nach Verdunstung des Wassers ab. Die Abtötung kann durch Zugabe einer antimikrobiellen Substanz zum porösen Verschlußstopfen oder durch Beschichtung des Verschlußstopfens damit unterstützt werden. Dieses offene System kann daher nur größere Flüssigkeitsverluste durch Verdunstung infolge des unterhalb der Zuspritzstelle angebrachten Ventiles vermeiden. Für alle Konnexionsstellen ohne diesen Schutz durch das Ventil ist das System ungeeignet, da unvorhersehbare Mengen an Flüssigkeit verdunsten können. Abgesehen hiervon ist das System auch nur zur Zuführung und nicht auch zur Entnahme von Flüssigkeiten geeignet, da das Ventil dieses verhindert. Schließlich gewährleistet das offene System keine dauernde keimhemmende Eigenschaft, da nicht ausgeschlossen werden kann, daß durch den porösen Verschlußstopfen Keime aufweisende Luft in den Stutzen gelangt.

Aus der DE-C-35 15 665 ist ein Verschlußelement zum dichten Verschließen eines Stutzens bei einem medizinischens Gerät bekannt. Das Verschlußelement ist als Stift ausgebildet, dessen freier Endbereich einen gegenüber dem Restbereich des Stiftes reduzierten Durchmesser aufweist, sodaß im Endbereich ein Radialspalt zwischen dem Stift und dem Stutzen gebildet ist. Der Spalt ist dort vorgesehen, um einen größeren Öffnungsspalt in der Offen-Stellung des Verschlußelements zu erhalten.

Es ist Aufgabe vorliegender Erfindung, das medizinische Gerät der genannten Art so weiter zu bilden, daß die Sterilität der im Gerät befindlichen Flüssigkeit insgesamt sichergestellt ist, das heißt auch die des Resttropfens, wobei hierunter selbstverständlich auch mehrere Tropfen zu verstehen sind. Das Gerät soll im Zuführ- und Entnahmebereich als geschlossenes System ausgebildet sein.

Gelöst wird die Aufgabe bei einem medizinischen Gerät der genannten Art dadurch, daß ein Radialspalt zwischen dem Stutzen und dem Stift bei mittels dem Verschlußelement dicht verschlossenem Stutzen vorgesehen ist.

Das erfindungsgemäße medizinische Gerät stellt sich bei verschlossenem Stutzen als geschlossenes System dar, so daß eine dauernde Sterilität bzw. Keimfreiheit gewährleistet ist. Der Stift gelangt bei verschlossenem Stutzen in Kontakt mit dem Resttropfen und wirkt aufgrund dessen keimhemmender bzw. keimtötender Eigenschaft als Keimsperre. Grundsätzlich wird jedes Stiftmaterial als geeignet angesehen, das biologisch verträglich ist. Vorzugsweise ist jedoch an antimikrobielle Wirkstoffe in Form von Metallen und Metallverbindungen gedacht, insbesondere solche, die eine oligodynamische Metallwirkung aufweisen, die bekannt ist. Verwiesen wird in diesem Zusammenhang auf Wallhäußer, Karl Heinz: Praxis der Sterilisation - Desinfektion - Konservierung - Keimidentifizierung - Betriebshygiene / 4., überarb. u. erw. Aufl. - Thieme, 1988, dort die Ziffern 6. und 7., insbesondere 7.26..

Vorteilhaft besteht der die keimhemmende bzw. keimtötende Eigenschaft aufweisende Stift aus Keramik, Glas, Metall oder Kunststoff mit einer oligodynamischen Metallwirkung aufweisenden Beschichtung, insbesondere einer Silberchloridbeschichtung. Es wird in diesem Zusammenhang als besonders vorteilhaft angesehen, wenn das Trägerelement für die Beschichtung in höchstem Maße porös ausgestaltet ist, womit sich eine Beschichtung über eine größere wirksame Oberfläche ergibt. Es ist jedoch auch denkbar, daß das die keimhemmende bzw. keimtötende Eigenschaft aufweisende Element vollständig aus einem, die oligodynamische Metallwirkung aufweisenden Metall besteht, beispielsweise aus Silber.

Eine besondere Ausführungsform der Erfindung sieht vor, daß das Verschlußelement als Verschlußstopfen ausgebildet ist, mit einem bei verschlossenem Stutzen teilweise in den Stutzen ragenden Stopfenansatz, bevorzugt in Art eines Verschluß-/Dichtkegels, der den Stift aufnimmt, der vorteilhaft in eine Axialbohrung des Stopfenansatzes eingesetzt ist. - Es ist allerdings auch möglich, daß der Stift und der Verschlußstopfen ein Bauteil bilden.

Zweckmäßig durchsetzt der Stift bei verschlossenem Stutzen diesen bis zu dessen dem Stopfen abgewandten Ende, wobei zwischen dem Stift und dem Stutzen ein Radialspalt verbleiben sollte. Beim Schließen des Stutzens verdrängt der Stift damit einen Teil des Resttropfens in den Radialspalt, womit der oligodynamische Effekt gesteigert werden kann, da der Resttropfen die gesamte zugängliche Oberfläche des Stiftes umschließt.
Eine Weiterbildung der Erfindung sieht vor, daß das Verschlußelement auf den Stutzen aufschraubbar ist, überdies sollte der Stutzen eine konische Stutzenöffnung und der Stopfenansatz des Verschlußstopfens entsprechend konisch ausgebildet sein, wobei die Konizität von Stutzenöffnung und Stopfenansatz insbesondere der des Kegelansatzes einer Injektionsspritze entsprechen sollte.

In den Figuren ist die Erfindung an einer Ausführungsform beispielsweise dargestellt, ohne auf diese beschränkt zu sein. Es zeigt:
- Figur 1: einen Schnitt durch den Anschluß eines medizinischen Gerätes zur Zuführung bzw. Entnahme von Flüssigkeiten für alle Arten von Injektions-, Infusions- oder Transfusionsanschlüssen oder sonstigen Entnahme- oder Zuführstellen bei Produkten der Medizintechnik, insbesondere Kunststoffeinmalprodukten, gezeigt mit Verschlußstopfen,
- Figur 2: einen Schnitt gemäß Figur 1, dargestellt ohne Verschlußstopfen, jedoch mit angeschraubter, teilweise dargestellter Spritze, und
- Figur 3: den Anschluß gemäß Figur 2 nach der Dekonnektion der Spritze.

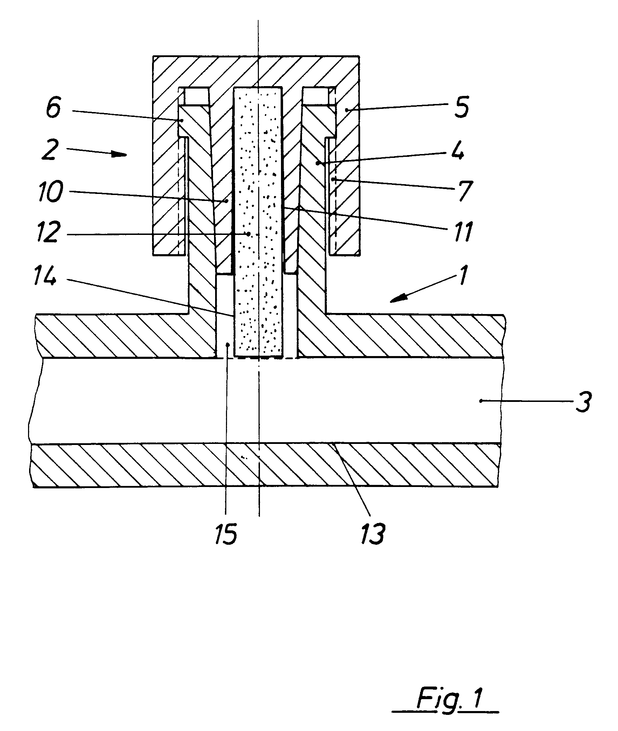

Figur 1 zeigt das medizinische Gerät im Bereich dessen erfindungswesentlichen Anschlusses. Das Gerät besteht dort im wesentlichen aus einem Gehäuse 1, einem Anschluß 2 mit Flüssigkeitskanal 3 sowie einem in den Anschluß 2 mündenden Zuführungs- bzw. Entnahmestutzen 4, der mittels eines Verschlußstopfens 5 dicht verschließbar ist.

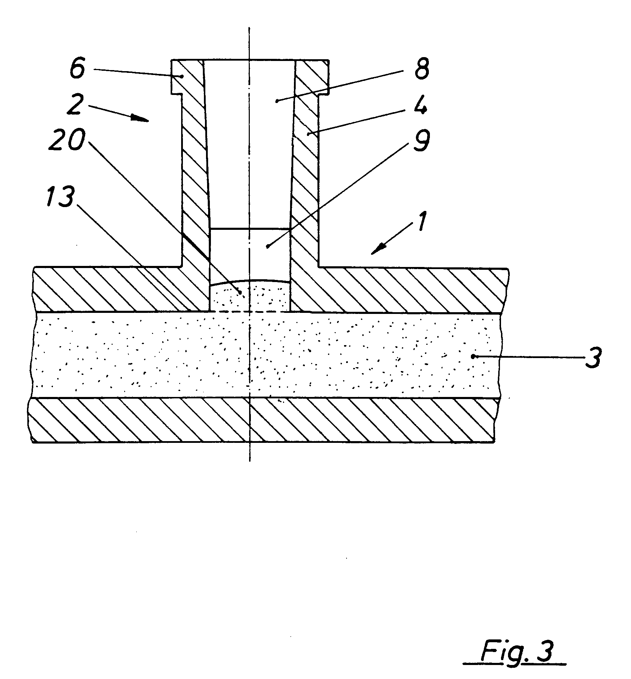

Wie in der Darstellung der Figur 1 veranschaulicht ist, weist der Stutzen 4 ein Außengewinde 6 und der Verschlußstopfen 5 ein Innengewinde 7 auf, womit die Möglichkeit besteht, den Verschlußstopfen 5 auf den Stutzen 4 aufzuschrauben. Desweiteren ist, wie insbesondere der Darstellung der Figur 3 zu entnehmen ist, die von dem Stutzen 4 umschlossene Öffnung in ihrem (bezogen auf die Darstellung der Figuren) oberen Bereich zugeordneten Öffnungsabschnitt 8 in Form eines sich nach unten verjüngenden Kegelstumpfes (weiblicher Luer-Lock-Ansatz) ausgebildet, an den sich zum Flüssigkeitskanal 3 hin ein zylindrischer Öffnungsabschnitt 9 anschließt. Entsprechend der Konizität des Öffnungsabschnittes 8 ist ein Stopfenansatz 10 des Verschlußstopfens 5 als Kegelstumpf (männlicher Luer-Lock-Kegel) ausgebildet, der in einer Axialbohrung 11 einen Stift 12 aufnimmt. Dieser ist in der Bohrung 11 durch eine Klemm-, Rast-Schweiß- oder Klebeverbindung dauerhaft gehalten. Der Stift 12 erstreckt sich bis zu dem dem Stutzen 4 zugewandten Innenwandungsbereich 13 des Flüssigkeitskanales 3 und weist Kreisquerschnitt auf, wobei zwischen dem aus dem Stopfenansatz 10 ragenden Stiftbereich 14 und der zugeordneten Innenwandung des Stutzens 4 ein Radialspalt 15 gebildet ist. Der Stift besteht aus Keramik und ist mit einer oligodynamischen Silberchloridbeschichtung versehen.

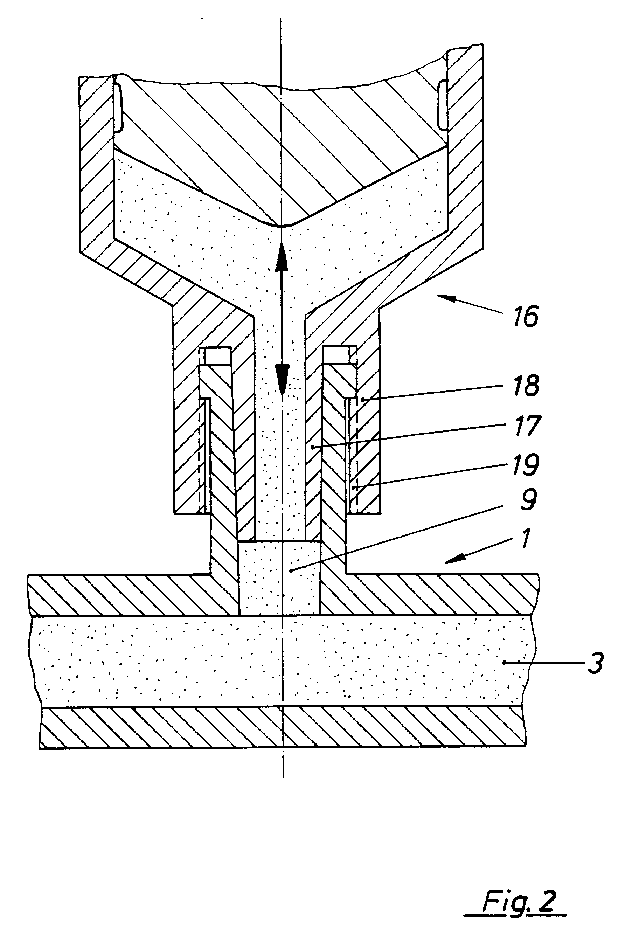

Figur 2 zeigt eine teilweise dargestellte Spritze 16 mit einem Kegelansatz 17, dessen Konizität der des Stopfenansatzes 10 entspricht und der gemäß der Ausbildung des Verschlußstopfens 5 mit einem Ansatz 18 mit Innengewinde versehen ist, so daß die Spritze, wie in dieser Figur gezeigt, dichtend mit dem medizinischen Gerät verbunden werden kann. Es ist damit eine Entnahme von Flüssigkeit aus dem medizinischen Gerät in die Spritze bzw. ein Zuführen von Flüssigkeit von der Spritze in das medizinische Gerät möglich. Nachdem solches erfolgt ist, wird die Spritze 16 vom Stutzen 4 abgeschraubt, nach der Dekonektion verbleibt, wie in der Darstellung der Figur 3 gezeigt, im Übergangsbereich vom Flüssigkeitskanal 3 zum Öffnungsabschnitt 9 des Stutzens 4 ein Resttropfen 20. Beim Aufschrauben des Verschlußstopfens 5 auf den Stutzen 4 verdrängt der Stift 12 mit seinem Stiftbereich 14 den Resttropfen 20, der in den Radialspalt 15 ausweicht und damit den Stiftbereich 14 vollständig umschließt und aufgrund der oligodynamischen Metallwirkung dessen Überzuges die Keimsperre wirksam wird.

Das Gehäuse 1 des medizinischen Gerätes und der Verschlußstopfen 5, das heißt auch der Stopfenansatz 10 bestehen vorzugsweise aus Kunststoff, so daß nur dem vom Stopfenansatz 10 aufgenommenen Stift 12, insbesondere nur dessen Überzug die oligodynamische Metallwirkung zukommt.

## Patentansprüche

1. Medizinisches Gerät zur Zuführung bzw. Entnahme von Flüssigkeiten mittels Injektion, Infusion oder Transfusion, das ein Gehäuse (1) mit einem Zuführ- bzw. Entnahmestutzen (4) sowie ein Verschlußelement (5) für den Stutzen (4) aufweist, wobei das Verschlußelement (5) mit einem keimhemmende bzw. keimtötende Eigenschaft aufweisenden Element (12) versehen ist, das bei verschlossenem Stutzen (4) in diesen ragt und das Element als Stift (12) ausgebildet ist, **dadurch gekennzeichnet**, daß ein Radialspalt (15) zwischen dem Stutzen (4) und dem Stift (12) bei mittels des Verschlußelementes (5) dicht verschlossenem Stutzen (4) vorgesehen ist.

2. Gerät nach Anspruch 1, **dadurch gekennzeichnet**, daß die keimhemmende bzw. keimtötende Eigenschaft des Stiftes (12) auf oligodynamischer Metallwirkung beruht.

3. Gerät nach Anspruch 2, **dadurch gekennzeichnet**, daß der Stift (12) vollständig aus einem oligodynamische Metallwirkung aufweisenden Metall, insbesondere aus Silber besteht.

4. Gerät nach Anspruch 2, **dadurch gekennzeichnet**, daß der Stift (12) aus Keramik, Glas, Metall oder Kunststoff besteht, mit einer oligodynamische Metallwirkung aufweisenden Beschichtung, insbesondere einer Silberchloridbeschichtung.

5. Gerät nach einem der Ansprüche 1 bis 4, **dadurch gekennzeichnet**, daß das Verschlußelement als Verschlußstopfen (5) ausgebildet ist, mit einem bei verschlossenem Stutzen (4) teilweise in den Stutzen (4) ragenden Stopfenansatz (10), der den Stift (12) aufnimmt.

6. Gerät nach einem der Ansprüche 1 bis 5, **dadurch gekennzeichnet**, daß der Stift (12) zumindest teilweise in eine Axialbohrung (11) des Stopfenansatzes (10) eingesetzt ist.

7. Gerät nach Anspruch 1 bis 6, **dadurch gekennzeichnet**, daß der Stift (12) bei verschlossenem Stutzen (4) diesen bis zu dessen dem Stopfen (5) abgewandten Ende durchsetzt.

8. Gerät nach einem der Ansprüche 1 bis 7, **dadurch gekennzeichnet**, daß der Stift (12) Kreisquerschnitt aufweist.

9. Gerät nach einem der Ansprüche 1 bis 8, **dadurch gekennzeichnet**, daß das Verschlußelement (5) auf den Stutzen (4) aufschraubbar ist.

10. Gerät nach einem der Ansprüche 1 bis 9, **dadurch gekennzeichnet**, daß der Stutzen (4) eine konische Stutzenöffnung (8) und der Stopfenansatz (10) des Verschlußstopfens (5) entsprechend konisch ausgebildet ist, wobei die Konizität von Stutzenöffnung (8) und Stopfenansatz (10) insbesondere der des Kegelansatzes (17) einer Injektionsspritze (16) entspricht.

## Claims

1. A medical appliance for the injection or extraction of fluids by way of injection, infusion or transfusion comprising a housing (1) with an injection or extraction socket (4) and a sealing element (5) for the socket (4), in which respect the sealing element (5) is provided with a germination inhibiting or germicidal element (12) which, when the socket (4) is sealed, intrudes into the latter and is designed as a pin (12) **characterized in that** a radial gap (15) is provided between the socket (4) and the pin (12) when the socket (4) is tightly sealed by means of the sealing element (5)

2. An appliance according to claim 1, **characterized in that** the germination inhibiting or germicidal property of the pin (12) is based on the oligodynamic metal effect.

3. An appliance according to claim 2, **characterized in that** the pin (12) is made entirely of a metal which offers an oligodynamic metal effect, in particular silver.

4. An appliance according to claim 2, **characterized in that** the pin (12) is made of ceramic, glass, metal or plastic material with a coating which offers an oligodynamic metal effect, in particular a silverchloride coating.

5. An appliance according to one of claims 1 to 4, **characterized in that** the sealing element is designed as a sealing plug (5), having a plug extension (10), which partially enters into the socket (4) when the socket (4) is sealed and which accommodates the pin (12).

6. An appliance according to one of claims 1 to 5, **characterized in that** the pin (12) is at least partially inserted into an axial bore (11) of the plug extension (10).

7. An appliance according to claim 1 to 6, **characterized in that** the pin (12) passes through the sealed socket (4) up to its end facing away from the plug (5).

8. An appliance according to one of claims 1 to 7, **characterized in that** the pin (12) is of circular cross-section.

9. An appliance according to one of claims 1 to 8, **characterized in that** the sealing element (5) can be screwed onto the socket (4).

10. An appliance according to one of claims 1 to 9, **characterized in that** the socket (4) comprises a conical socket aperture (8), and the plug extension (10) of the sealing plug (5) is of respective conical shape, and that the conicity of socket aperture (8) and plug extension (10) corresponds in particular with that of the conical extension (17) of a syringe (16).

## Revendications

1. Appareil médical pour introduire ou prélever des liquides par injection, par perfusion ou par transfusion, et qui comporte un boîtier (1) ayant une tubulure d'entrée et une tubulure de prélèvement (4) ainsi qu'un élément de fermeture (5) pour la tubulure (4), l'élément de fermeture (5) étant muni d'un élément (12) à propriété d'inhibition des germes ou germicide et qui, lorsque la tubulure (4) est fermée, pénètre dans cette dernière, l'élément étant réalisé sous la forme d'une tige (12), caractérisé par le fait qu'un intervalle radial (15) est prévu entre la tubulure (4) et la tige (12) lorsque la tubulure (4) est fermée de façon étanche au moyen de l'élément de fermeture (5).

2. Appareil suivant la revendication 1, caractérisé par le fait que la caractéristique d'inhibition des germes ou germicide de la tige (12) repose sur une action oligodynamique d'un métal.

3. Appareil suivant la revendication 2, caractérisé par le fait que la tige (12) est entièrement en un métal, notamment en argent, qui possède une action oligodynamique.

4. Appareil suivant la revendication 2, caractérisé par le fait que la tige (12) est en céramique, en verre, en métal ou en matière plastique et comporte un revêtement qui possède une action oligodynamique, notamment un revêtement en chlorure d'argent.

5. Appareil suivant l'une des revendications 1 à 4, caractérisé en ce que l'élément de fermeture est agencé sous la forme d'un bouchon de fermeture (5), comportant un embout (10) qui pénètre en partie dans la tubulure (14) lorsque cette dernière est fermée et reçoit la tige (12).

6. Appareil suivant l'une des revendications 1 à 5, caractérisé par le fait que la tige (12) est insérée au moins partiellement dans un perçage axial (11) de l'embout (10) du bouchon.

7. Appareil suivant les revendications 1 à 6, caractérisé par le fait que lorsque la tubulure (14) est fermée, la tige (12) traverse cette tubulure jusqu'à son extrémité tournée à l'opposé du bouchon (5).

8. Appareil suivant l'une des revendications 1 à 7, caractérisé par le fait que la tige (12) a une section transversale circulaire.

9. Appareil suivant l'une des revendications 1 à 8, caractérisé par le fait que l'élément de raccordement (5) peut être vissé sur la tubulure (14).

10. Appareil suivant l'une des revendications 1 à 9, caractérisé par le fait que la tubulure (4) a une ouverture conique (8) et que l'embout (10) du bouchon de fermeture (5) a une forme conique correspondante, la conicité de l'ouverture (8) de la tubulure et de l'embout (10) du bouchon correspondant notamment à celle de l'embout conique (17) d'une seringue d'injection (16).
